# EUROPEAN PATENT APPLICATION

(11) **EP 2 272 541 A1**
(43) Date of publication of application: **12.01.2011**
(21) Application number: 09713864.8
(22) Date of filing: 27.02.2009
(51) Int. Cl.: A61L 9/01

(54) **AROMATIC AGENT COMPOSITION FOR OPENABLE STORAGE SPACE**

(30) Priority: 27.02.2008 JP 2008046938
(71) Applicant: Kobayashi Pharmaceutical Co., Ltd., Chuo-ku Osaka-shi Osaka 541-0045 (JP)
(72) Inventor: NISHI, Yumiko, Ibaraki-shi Osaka 567-0057 (JP)
(74) Representative: Wiedemann, Peter
(86) International application number: PCT/JP2009/053767
(87) International publication number: WO 2009/107813

(57) **Abstract**

An object of the present invention is to provide an aromatic agent composition to be used in an openable storage space that is capable of maintaining a constant and homogeneous aroma intensity in a closed storage space, and capable of maintaining aroma emission at a constant and homogeneous intensity in a storage space that is occasionally opened and closed.

The present invention uses, as a solvent of an aromatic agent composition, a glycol ether having a boiling point of 120 to 185°C under ordinary pressure so that the glycol ether is present in an amount of 15 to 90 wt%.

## Description

### [Technical Field]

The present invention relates to an aromatic agent composition to be used in openable storage spaces, and a production process thereof. More specifically, the present invention relates to an aromatic agent composition to be used in openable storage spaces that is capable of emitting a homogeneous aroma to storage spaces at a constant intensity, and a production process thereof. The present invention further relates to a process for emitting a homogeneous aroma to storage spaces at a constant intensity. The present invention still further relates to a process for adjusting aroma emission to an openable storage space to a constant and homogeneous intensity.

### [Background Art]

Aromatic agents are widely used to eliminate unpleasant odors from storage spaces or rooms, thereby keeping the air in indoor spaces fresh and comfortable.

Generally, the volatility rates of aromatic agents are not high enough for the large capacities of rooms. Therefore, there has been a demand to increase the volatilities of the fragrance materials of aromatic agents so that the aroma can be continuously emitted to large spaces.

On the other hand, the volatility rates of the fragrance materials of aromatic agents are often overly high for storage spaces such as drawers, footwear cupboards or the like, whose capacities are usually smaller than rooms. Moreover, cupboards and drawers are kept closed most of the time, except when objects are stored or taken out. Therefore, if a fragrance material with an increased volatility is used in a storage space so that the aroma is continuously emitted, the aroma emission amount in the storage space may become overly high, and the aroma perceived by the users when they open the storage space may become overly strong. Additionally, the aromatic agents placed in storage spaces are also used to transfer the aroma to the objects stored therein; therefore, if the aroma emission amount in the storage space becomes overly high, the aroma transferred to the objects becomes overly strong. Moreover, the increase in the aroma emission amount in the closed storage space may result in inhomogeneous intensity of the aroma depending on the length of the period in which the storage space is closed, and may further shorten the usable period of the aromatic agent. Therefore, ensuring a constant and homogeneous intensity of the aroma is indispensable for the aromatic agents used in openable storage spaces. This characteristic is more important than increasing the volatility and aroma-emitting duration of the aromatic agent, which is required for the aromatic agent used in rooms.

In recent years, various types of aromatic agents have been developed and commercialized. However, research has not achieved the production of an aromatic agent that is capable of maintaining a constant and homogeneous aroma intensity in a closed storage space, and providing aroma emission at a constant and homogeneous intensity in a storage space that is occasionally opened and closed.
[Patent Literature 1] Japanese Unexamined Patent Publication No. 2003-102822

### [Disclosure of Invention]

### [Technical Problem]

An object of the present invention is to provide an aromatic agent composition for an openable storage space that is capable of maintaining a constant and homogeneous aroma intensity in a closed storage space, and capable of maintaining aroma emission at a constant and homogeneous intensity in a storage space that is occasionally opened and closed. Another object of the present invention is to provide a process for emitting a homogeneous aroma to an openable storage space at a constant intensity. Still another object of the present invention is to provide a process for adjusting a volatility of an aroma emitted to an openable storage space at a constant and homogeneous intensity.

### [Solution to Problem]

The inventors of the present invention conducted intensive studies to achieve the above objectives, and found that an aromatic agent composition capable of maintaining the aroma at a homogeneous and constant intensity in a closed storage space can be obtained by using a glycol ether having a boiling point of 120 to 185°C under ordinary pressure as a solvent of the fragrance material, so that the glycol ether is present in an amount of 15 to 90% by weight. The inventors confirmed that an aromatic agent composition having this constitution is capable of providing aroma emission of constant and homogeneous intensity to a storage space that is occasionally opened and closed. The inventors conducted further research based on these findings, and completed the present invention.

Specifically, the present invention provides the following embodiments based on the aforementioned findings.
Item 1. An aromatic agent composition for an openable storage space, comprising a fragrance material and 15 to 90 wt% of a glycol ether having a boiling point of 120 to 185°C under ordinary pressure.
Item 2. The aromatic agent composition according to Item 1, wherein the glycol ether is present in an amount of 25 to 55 wt% of the glycol ether.
Item 3. The aromatic agent composition according to Item 1, wherein the glycol ether has a vapor pressure of 0.05 to 1 kPa at 20°C.
Item 4. The aromatic agent composition according to Item 1, wherein the glycol ether is a monopropylene glycol monoalkyl ether.
Item 5. The aromatic agent composition according to Item 1, wherein the glycol ether is a monopropylene glycol mono-n-propyl ether.
Item 6. The aromatic agent composition according to Item 1, wherein the fragrance material is present in an amount of 10 to 85 wt%.
Item 7. The aromatic agent composition according to Item 1, wherein the fragrance material has a boiling point of 50 to 360°C under ordinary pressure.
Item 8. The aromatic agent composition according to Item 1, wherein the fragrance material is at least one member selected from the group consisting of d-limonene, caryophyllene, cis-3-hexenol, geraniol, phenylethyl alcohol, diphenyloxide, dl-roseoxide, hexanal, hexyl cinnamic aldehyde, ethyl butyrate, styralyl acetate, o-t-butylcyclohexyl acetate, 2-octanone and 5-damascone.
Item 9. The aromatic agent composition according to Item 1, wherein the fragrance material is present in an amount of 45 to 75 wt%, and the glycol ether is present in an amount of 25 to 55 wt%.
Item 10. The aromatic agent composition according to Item 1, wherein the aromatic agent composition is a liquid.
Item 11. The aromatic agent composition according to Item 1, wherein the aromatic agent composition is placed in a drawer or a footwear cupboard.
Item 12. The aromatic agent composition according to Item 1, wherein the aromatic agent composition is placed in a clothing storage space.
Item 13. A process for producing an aromatic agent composition for an openable storage space, comprising:
   mixing a fragrance material and a glycol ether having a boiling point of 120 to 185°C under ordinary pressure so that the glycol ether is present in an amount of 15 to 90 wt%.
Item 14. A process for emitting an aroma to an openable storage space, comprising:
   emitting an aromatic agent composition comprising a fragrance material and 15 to 90 wt% of a glycol ether having a boiling point of 120 to 185°C under ordinary pressure, to an openable storage space.
Item 15. A process for adjusting a volatility of an aroma emitted to an openable storage space, comprising:
   mixing a fragrance material and a glycol ether having a boiling point of 120 to 185°C under ordinary pressure so that the glycol ether is present in an amount of 15 to 90 wt%.
Item 16. Use of a composition comprising a fragrance material and 15 to 90 wt% of a glycol ether having a boiling point of 120 to 185°C under ordinary pressure, for producing an aromatic agent composition for an openable storage space.
Item 17. Use of a glycol ether having a boiling point of 120 to 185°C under ordinary pressure, for adjusting a volatility of a fragrance material in an openable storage space, the glycol ether being present in an amount of 15 to 90 wt% with respect to a gross amount of an aromatic agent composition.

### [Advantageous Effects of Invention]

The present invention provides, to a fragrance material of the aromatic agent composition, a volatility characteristic required in openable storage spaces.

More specifically, the aromatic agent composition of the present invention is capable of maintaining the aroma at a constant and homogeneous intensity in closed storage spaces, and is also capable of maintaining such constant and homogeneous aroma emission to the storage spaces even when the storage spaces are repeatedly opened and closed. Therefore, by placing the aromatic agent composition of the present invention in storage spaces, such as drawers, footwear cupboards or the like, it is possible to fill the storage spaces with a desired aroma, and transfer the aroma to the objects stored therein.

### [Best Mode for Carrying Out the Invention]

### I. Aromatic agent composition

The aromatic agent composition of the present invention is used in openable storage spaces. The aromatic agent composition is characterized by containing a fragrance material, and 15 to 90 wt% of a glycol ether having a boiling point of 120 to 185°C under ordinary pressure. The following describes an embodiment of the aromatic agent composition of the present invention in detail.

Examples of the fragrance materials contained in the aromatic agent composition of the present invention include natural fragrance materials, aroma chemicals isolated from the natural fragrance materials, artificial aroma chemicals, and blended fragrance materials created by mixtures of these fragrance materials. They can be selected from various existing fragrance materials. Specifically, examples of the aroma chemicals include hydrocarbon-based fragrance materials such as d-limonene, caryophyllene, α-pinene, β-pinene, myrcene, terpinolene, ocimene, γ-terpinene, α-phellandrene, p-cymene, β-caryophyllene, β-farnesene, 1,3,5-undecatriene, and diphenylmethane; alcohol-based fragrance materials such as cis-3-hexenol, linalool, geraniol, phenyl ethyl alcohol, trans-2-hexenol, cis-3-hexenol, 3-octanol, 1-octen-3-ol, 2,6-dimethyl-2-heptanol, 9-decenol, 4-methyl-3-decene-5-ol, 10-undecenol, trans-2-cis-6-nonadiethanol, linalool, geraniol, nellore, citronellol, rhodinol, myrcenol, lavandulol, tetrahydrogeraniol, tetrahydro linalool, hydroxy citronellol, dihydromyrcenol, aloxymethanol, terpineol, α-terpineol, terpinene-4-ol, 1-menthol, borneol, isopulegol, nopol, farnesol, nerolidol, cedrol, patchouli alcohol, vetivelol, 2,4-dimethyl-3-cyclohexene-1-methanol, 4-isopropylcyclohexanol, 4-isopropylcyclohexanemethanol, 1-(4-isopropylcyclohexyl)-ethanol, 2,2-dimethyl-3 -(3-methylphenyl)-propanol, p-t-butylcyclohexanol, o-t-butylcyclohexanol, ambrinol, 1-(2-t-butylcyclohexyloxy)-2-butanol, pentamethylcyclohexylpropanol, 1-(2,2,6-trimethyl cyclohexyl)-3-hexanol, benzyl alcohol, phenyl ethyl alcohol, phenoxyethyl alcohol, styralyl alcohol, anise alcohol, cinnamic alcohol, phenylpropyl alcohol, dimethylbenzyl carbinol, dimethylphenylethyl carbinol, phenylethylmethylethyl carbinol, 3-methyl-5-phenylpentanol, thymol, carvacrol, orcinol monomethyl ether, eugenol, isoeugenol, propenyl guaethol, santalol, isobornyl cyclohexanol, sandalore, bagdanol, sandal mysore core, brahmanol, ebanol, polysantol, and 3,7-dimethyl-7-methoxy octan-2-ol; ether-based fragrance materials such as diphenyloxide, p-cresyl ethylether, dl-rose oxide (nellore oxide, myroxyde, 1,8-cineole, rose oxide, limetol, menthofuran, linalool oxide, butyldimethyldihydroxy piran, acetoxyamyl tetrahydropyran, credrol methyl ether, methoxy cyclododecane, 1-methyl-1-methoxy cyclododecane, ethoxymethyl cyclododecyl ether, trichlodecenyl methyl ether, rhubofix, cedroxide, ambroxan, grisalva, boisiris, anisole, dimethylhydroquinone, para-cresyl methyl ether, acetanisole, anethole, dihydroanethole, estragole, diphenyl oxide, methyleugenol, phenylethyl isoamyl ether, β-naphthol methyl ether, and β-naphthyl isobutyl ether; aldehyde fragrance materials such as hexanal, citral, hexyl cinnamic aldehyde, hexyl aldehyde, octyl aldehyde, nonyl aldehyde, decyl aldehyde, undecyl aldehyde, dodecyl aldehyde, tridecyl aldehyde, trimethylhexyl aldehyde, methyloctylacetyl aldehyde, methylnonylacet aldehyde, trans-2-hexenal, cis-4-heptenal, 2,6-nonadienal, cis-4-decenal, trans-4-decenal, undecylene aldehyde, trans-2-dodecenal, trimethyl undecenal, 2,6,10-trimethyl-5,9-undecadienal, citronellal, hydroxycitronellal, perilaldehyde, methoxy dihydrocitronellal, citronellyl oxyacetaldehyde, 2,4-dimethyl-3-cyclohexenyl carboxy aldehyde, isocyclocitral, scentenal, myrac aldehyde, lyral, vernaldehyde, dupicall, maceal, boronal, cetonal, benzaldehyde, phenylacet aldehyde, phenylpropyl aldehyde, cinnamic aldehyde, α-amyl cinnamic aldehyde, α-hexylcinnamic aldehyde, hydrotropic aldehyde, anis aldehyde, p-methylphenyl acet aldehyde, cumin aldehyde, cyclamen aldehyde, 3-(p-t-butylphenyl)-propyl aldehyde, p-ethyl-2,2-dimethylhydrocinnam aldehyde, 2-methyl-3-(p-methoxyphenyl)-propyl aldehyde, p-t-butyl-α-methylhydrocinnamic aldehyde, salicyl aldehyde, heliotropin, helional, vanillin, ethyl vanillin, and methyl vanillin; acetal fragrance materials such as octyl aldehyde glycol acetal, acetaldehyde ethyl cis-3-hexenyl acetal, citral dimethyl acetal, citral diethyl acetal, acetaldehyde ethyl linalyl acetal, acetaldehyde ethyl linalyl acetal, hydroxycitronellal dimethyl acetal, phenyl acetaldehyde dimethyl acetal, hydratropic aldehyde dimethyl acetal, phenyl acetaldehyde glyceryl acetal, acetaldehyde ethyl phenyl acetal, acetaldehyde phenyl ethyl propyl acetal, phenylpropyl aldehyde propylene glycol acetal, 4,4,6-trimethyl-2-benzyl-1,3-dioxane, 2,4,6-trimethyl-2-phenyl-1,3-dioxane, 2-butyl-4,4,6-trimethyl-1,3-dioxane, tetrahydroindeno-m-dioxin, dimethyltetrahydroindeno-m-dioxin, and caranal; ester fragrance materials such as ethyl butyrate, styralyl acetate, o-t-butylcyclohexyl acetate, ethyl formate, cis-3-hexenyl formate, linalyl formate, citronellyl formate, geranyl formate, benzyl formate, phenylethyl formate, ethyl acetate, butyl acetate, isoamyl acetate, methyl cyclopentylidene acetate, hexyl acetate, cis-3-hexenyl acetate, trans-3-hexenyl acetate, isononyl acetate, citronellyl acetate, lavandulyl acetate, geranyl acetate, linalyl acetate, myrcenyl acetate, terpinyl acetate, mentyl acetate, mentanyl acetate, nopyl acetate, n-bornyl acetate, isobornyl acetate, p-t-butylcyclohexyl acetate, o-t-butylcyclohexyl acetate, tricyclodecenyl acetate, 2,4-dimethyl-3-cyclohexene-1-methanyl acetate, benzyl acetate, phenylethyl acetate, stryralyl acetate, cinnamyl acetate, anicyl acetate, para-cresyl acetate, heliotropyl acetate, acetyl eugenol, acetyl isoeugenol, guayl acetate, cedolyl acetate, betiberyl acetate, decahydro-β-naphthyl acetate, ethyl propionate, isoamyl propionate, citronellyl propionate, geranyl propionate, linalyl propionate, terpinel propionate, benzyl propionate, cinnamyl propionate, allyl cyclohexylpropionate, tricyclodecenyl propionate, ethyl butyrate, ethyl-2-methylbutyrate, butyl butyrate, isoamyl butyrate, hexyl butyrate, linalyl butyrate, geranyl butyrate, citronellyl butyrate, benzyl butyrate, cis-3-hexenyl isobutyrate, citronellyl isobutyrate, geranyl isobutyrate, linalyl isobutyrate, benzyl isobutyrate, phenylethyl isobutyrate, phenoxyethyl isobutyrate, tricyclodecenyl isobutyrate, ethyl valerate, propyl valerate, citronellyl isovalerate, geranyl isovalerate, cinnmamyl isovalerate, benzyl isovalerate, phenylethyl isovalerate, ethyl capronate, allyl capronate, ethyl enantoate, allyl enantoate, ethyl caprylate, citronellyl tiglate, methyl octinoate carboxylate, allyl 2-pentyloxyglycolate, cis-3-hexenylmethyl carbonate, keto acid ethyl, isoamyl pyruvate, acetoacid ethyl, ethyl levulinate, methyl benzoate, ethyl benzoate, isobutyl benzoate, isoamyl benzoate, geranyl benzoate, linalyl benzoate, benzyl benzoate, phenylethyl benzoate, phenylethyl benzoate, methyl dihydroxymethyl benzoate, methyl phenylacetate, methyl phenylacetate, ethyl phenylacetate, isobutyl phenylacetate, isoamyl phenylacetate, geranyl phenylacetate, benzyl phenylacetate, phenylethyl phenylacetate, p-cresyl phenylacetate, methyl cinnamate, ethyl cinnamate, benzyl cinnamate, cinnamyl cinnamate, phenylethyl cinnamate, methyl salicylate, ethyl salicylate, isobutyl salicylate, isoamyl salicylate, hexyl salicylate, cis-3-hexenyl salicylate, benzyl salicylate, phenylethyl salicylate, methyl anisate, ethyl anisate, methyl anthranylate, ethyl anthranylate, methyl methylanthranylate, methyl jasmonate, methyl dihydrojasmonate, ethyl methylphenylglycidate, ethyl phenylglycidate, glycomel, fracton, freistone, fretate, dibescone, and ethyl-2-methyl-6-pentyl-4-oxa-2-cyclohexenecarbonate; ketone fragrance materials such as 2-octanone, δ-damascone, acetoin, diacetyl, methyl amyl ketone, ethyl amyl ketone, methyl hexyl ketone, methyl nonyl ketone, methylheptenone, koavone, camphor, carvone, menthone, d-pulegone, piperitone, fenchone, geranyl acetone, cedryl methyl ketone, nootkatone, ionone, α-ionone, β-ionone, methyl ionone, α-n-methyl ionone, β-n-methyl ionone, α-iso ionone, β-iso ionone, allyl ionone, irone, α-irone, β-irone, γ-irone, damascone, α-damascone, β-damascone, δ-damascone, damascenone, dynascone, α-dynascone, β-dynascone, maltol, ethyl maltol, 2,5-dimethyl-4-hydroxyfuranone, sugar lactone, p-t-butylcyclohexanone, amyl cyclopentanone, heptylcyclopentanone, dihydrojasmone, cis-jasmone, florex, plicatone, 4-cyclohexyl-4-methyl-2-pentanone, p-menthene-6-ylpropanone, 2,2,5-trimethyl-5-pentylcyclopentanone, ethoxyvinyltetracyclohexanone, dihydropentamethylindanon, Iso E Super, trimofix, acetophenone, p-methylacetophenone, benzylacetone, calone, raspberry ketone, anisylacetone, 4-(4-hydroxy-3-methoxy phenyl)-2-butanone, methyl naphthyl ketone, 4-phenyl-4-methyl-2-pentanone and benzophenone; carboxylic acid fragrance materials such as geranyl acid, citronellylic acid, benzoic acid, phenylacetate, phenylpropionic acid, cinnamic acid and 2-methyl-2-pentenoic acid; lactone fragrance materials such as γ-octalactone, γ-nonalactone, γ-decalactone, γ-undecalactone, δ-decalactone, coumarin, dihydrocoumarin, jasmolactone, and jasmine lactone; musk fragrance materials such as muscone, civetone, cyclopentadecanone, cyclohexadecenone, cyclopentadecanolide, 12-keto cyclopentadecanolide, cyclohexadecanolide, cyclohexadecenolide, 12-oxa-16-hexadecanolide, 11-hexa-16-hexadecanolide, 10-oxa-16-hexadecanolide, ethylene brassylate, ethylenedodecane dioate, musk ketone, musk xylene, musk ambrette, musk tibetene, musk moskene, 6-acetylhexamethylindan, 4-acetyldimethyl-t-butylindan, 5-acetyltetramethyl isopropylindan, 6-acetylhexatetralin and hexamethylhexa hydro cyclopentane benzopyran; nitrogen-containing fragrance materials such as acetylpyrrole, indore, skatole, indolene, 2-acetylpyridine, *Armeria maritime,* 6-methylquinoline, 6-isopropylquinoline, isobutylquinoline, 2-acetyl pyrazine, 2,3-dimethyl pyrazine, 2-isopropyl-3-methoxy pyrazine, 2-isobutyl-3-methoxy pyrazine, 2-secondary butyl-3-methoxy pyrazine, trimethyl pyrazine, 5-methyl-3-heptanoxime; nitrile fragrance materials such as geranyl nitrile, citronellyl nitrile, 5-phenyl-2,6-nonadienenitrile, cinnamon nitrile, cumin nitrile, dodecanenitrile, tridecene-2-nitrile; and sulfur-containing fragrance materials such as dimethylsulfide, 2-methyl-4-propyl-1,3-oxathiane, allyl isocyanate, p-menthan-8-thiol-3-one, p-menthene-8-thiol and p-methyl-menthylthio propionate. Examples of natural fragrance materials include tuberose oil, musk tincture, castoreum tincture, civet tincture, ambergris tincture, peppermint oil, perilla oil, petitgrain oil, pine oil, rose oil, rosemary oil, camphor oil, ho oil, clary sage oil, sandalwood oil, spearmint oil, spike lavender oil, star anise oil, lavandin oil, lavender oil, lemon oil, lemon grass oil, lime oil, neroli oil, oakmoss oil, ocotea oil, patchouli oil, thyme oil, tonka bean tincture, turpentine oil, vanilla beans tincture, basil oil, nutmeg oil, citronella oil, clove oil, bois de rose oil, cananga oil, cardamom oil, cassia oil, cedarwood oil, orange oil, mandarin oil, tangerine oil, aniseed oil, bay oil, coriander oil, elemi oil, eucalyptus oil, fennel oil, galbanum oil, geranium oil, hiba oil, hinoki oil, jasmine oil, vetiver oil, bergamot oil, ylang ylang oil, grapefruit oil and yuzu oil.

These fragrance materials may be used for the aromatic agent composition of the present invention solely or as a blended fragrance of plural materials.

A particularly preferred material among the above-listed fragrance materials is a substance having a boiling point of 50 to 360°C, more preferably 70 to 340°C, further preferably 90 to 310°C under ordinary pressure. When using a blended fragrance, it is not necessary that each fragrance material contained in the mixture have the above-specified boiling point, insofar as the resulting blended fragrance has the above-specified boiling point. By using the above-specified fragrance materials, it is possible to further increase the effect of the present invention, i.e., the effect of maintaining an aroma emitted to a closed storage space at a homogeneous and constant intensity.

In the present invention, preferable examples of the fragrance materials include d-limonene, caryophyllene, cis-3-hexanol, geraniol, phenylethyl alcohol, diphenyloxide, dl-roseoxide, hexanal, hexyl cinnamic aldehyde, ethyl butyrate, styralyl acetate, o-t-butylcyclohexyl acetate, 2-octanone, and delta-damascone. These fragrance materials further emphasize the required volatility characteristic of the aromatic agent composition of the present invention to be used in storage spaces.

The proportion of the fragrance material in the aromatic agent composition of the present invention cannot be uniformly defined, as it depends on the type of the fragrance materials used, the expected aromatic effects etc. As one exemplary proportion, the total amount of the fragrance material is 10 to 85 wt%, preferably 25 to 85 wt%, further preferably 45 to 75 wt%, with respect to the gross weight of the aromatic agent composition.

In addition to the fragrance material, the aromatic agent composition of the present invention contains a glycol ether having a boiling point of 120 to 185°C, more preferably 130 to 170°C, further preferably 140 to 160°C, under ordinary pressure. By using the specific glycol ether as a solvent at the proportion specified later, it is possible to further emphasize the required volatility characteristic of the fragrance material to be used in storage spaces.

Specific examples of glycol ethers having a boiling point within the above-mentioned range include monoethylene glycol monoalkyl ethers, such as monoethylene glycol monomethyl ether, mono-ethylene glycol monoethyl ether, monoethylene glycol monoisopropyl ether, monoethylene glycol mono-n-butyl ether, monoethylene glycol monoisobutyl ether, and monoethylene glycol monoaryl ether; monoethylene glycol dialkyl ethers, such as monoethylene glycol dimethyl ether; diethylene glycol dialkyl ethers, such as diethylene glycol methyl ethyl ether and diethylene glycol diethyl ether; monopropylene glycol monoalkyl ethers, such as monopropylene glycol monomethyl ether, mono propylene glycol monoethyl ether, mono propylene glycol monopropyl ether and monopropylene glycol mono-n-butyl ether; dipropylene glycol dialkyl ethers, such as dipropylene glycol dimethyl ether; monoethylene glycol monoalkyl ether acetate, such as monoethylene glycol monomethyl ether acetate; and monopropylene glycol monoalkyl ether acetates, such as monopropylene glycol monomethyl ether acetate. Among them, in view of further enhancing the effect of the present invention of ensuring homogeneous and constant intensity of the aroma emitted in the closed storage space, it is preferable to use as the above-specified glycol ether monopropylene glycol monoalkyl ether or monopropylene glycol monoalkyl ether acetate; more preferably, monopropylene glycol monoalkyl (C2 or C3) ether, and monopropylene glycol monoalkyl (C1 or C2) ether acetate; particularly preferably, monopropylene glycol mono-n-propyl ether.

In addition to meeting the specific range of boiling points, the glycol ether contained in the aromatic agent composition of the present invention preferably has a vapor pressure of 0.05 to 1 kPa, preferably 0.08 to 0.8 kPa, more preferably 0.1 to 0.6 kPa at 20°C. The glycol ethers with the specific vapor pressure further increase the effect of the present invention, i.e., the effect of maintaining an aroma emitted to a closed storage space at a homogeneous and constant intensity.

To be used for the aromatic agent composition of the present invention, the glycol ether having the specific boiling point may be used solely or as an arbitrary combination.

To ensure the required volatility characteristic of the fragrance material contained in the aromatic agent composition to be used in a storage space, the proportion of the glycol ether having the specific boiling point in the aromatic agent composition of the present invention falls within the range of from 15 to 90 wt%. The proportion of the glycol ether having the above-specified boiling point in the aromatic agent composition of the present invention is preferably 15 to 75 wt%, more preferably 25 to 55 wt%.

In addition to the fragrance material and the glycol ether, the aromatic agent composition of the present invention may contain other ingredients to an extent that the effects of the present invention are not impaired. Examples of the other ingredients to be contained in the aromatic agent composition of the present invention include surfactants, antiseptics, antioxidants, solvents, deodorizers, disinfection agents, UV absorbers, pH adjusters, insecticidal components, mothproof components, and repellents.

Examples of surfactants suitable for the aromatic agent composition of the present invention include nonionic surfactants, such as polyoxyethylene hydrogenated castor oil, polyoxyethylene hydrogenated castor oil ether, polyoxyethylene alkyl ether, polyoxyethylene lauryl ether, polyoxyethylene oleyl ether, polyoxyethylene fatty acid ester, polyoxyethylene glyceryl ether fatty acid ester, alkyl alkanolamide, alkyl polyglucoside, sorbitan fatty acid ester, glycerine fatty acid ester, polyoxyethylene sorbitan fatty acid ester, and polyoxyethylene polyoxypropylene glycol; anionic surfactants, such as alkyl sulfate, polyoxyethylene alkyl ether sulfate, sulfosuccinate, N-acylamino acid salt, carboxylate, sulfonic acid, and phosphoric ester; cationic surfactants, such as alkylammonium salt; and amphoteric surfactants such as alkyl amido betaine, and alkyl dimethylamine oxide.

Examples of preservatives suitable for the aromatic agent composition of the present invention include sorbic acid, methyl p-oxybenzoate, 1,2-benzisothiazolone-3-one, and 2n-octyl-isothiazolone-3-one.

Examples of antioxidants suitable for the aromatic agent composition of the present invention include dibutylhydroxytoluene (BHT), butylated hydroxyanisole (BHA), ascorbic acid salts, isoflavone, and α-tocopherol.

Examples of solvents suitable for the aromatic agent composition of the present invention include water, 3-methoxy-3-methyl-1-butanol, light liquid isoparaffin, ethanol, propanol, butanol, ethylene glycol, propylene glycol, and dipropylene glycol.

Examples of deodorizers suitable for the aromatic agent composition of the present invention include dichloroisocyanuric acid salts; plant extracts such as, rice, pine, cypress, bamboo grass, persimmon and tea; desalted betaine compounds; modified organic acid compounds; alkanolamines; stabilized chlorine dioxides; aldehyde compounds; glycol ether compounds; phytoncide-based perfumes; and lower aliphatic aldehyde perfumes.

Examples of disinfection agents suitable for the aromatic agent composition of the present invention include isopropylmethyl phenol, parahydroxybenzoic acid ester, PCMX, IPBC, chlorhexidine gluconate, chlorhexidine hydrochloride, benzethonium chloride, cetylpyridinium chloride, chlorine dioxide, dichloroisocyanuric acid salts, terpene hydrocarbon perfumes, terpene alcohol-based perfumes, phenol-based perfumes, aromatic alcohol-based perfumes, and aldehyde-based perfumes.

Examples of UV absorbers suitable for the aromatic agent composition of the present invention include benzotriazole-based UV absorbers (2-(3,5-di-tert-pentyl-2-hydroxyphenyl)-2H-benzotriazole) and benzophenone-based UV absorbers (2,2 4,4 tetrahydroxybenzophenone).

Examples of pH adjusters suitable for the aromatic agent composition of the present invention include alkanolamines such as monoethanolamine, diisopropanolamine, diethanolamine, and triethanolamine; citric acid alkali metal salts such as trisodium citrate and potassium citrate; calcium salts such as ethylenediaminetetraacetic acid, sodium hydroxide, basic amino acid (arginine), and calcium carbonate.

Examples of pH adjusters suitable for the aromatic agent composition of the present invention include hinokitiol, hiba oil, allylisothiocyanate, propylene glycol monomethyl ether, ethanol, propanol, and 1.8-cineol.

Examples of mothproof components suitable for the aromatic agent composition of the present invention include pyrethroid-based compounds, naphthalene-based compounds, paradichlorobenzene-based compounds, and camphor.

Examples of repellents suitable for the aromatic agent composition of the present invention include N,N-diethyl-m-toluamide, dimethyl phthalate, dibutyl phthalate, p-menthane-3,8-diol, 2-ethyl-1,3-hexanediol, di-n-propylisocinchomeronate, p-dichlorobenzene, di-n-butyl succinate, carane-3,4-diol, 1-methylpropyl-2-(2-hydroxyethyl)-1-piperidine carboxylate, allyl isothiocyanate, terpene-hydrocarbon-based perfumes, terpene alcohol-based perfumes, phenol-based perfumes, aromatic alcohol-based perfumes, aldehyde-based perfumes, ketone-based perfumes, plant extracts such as mustard, Japanese horseradish, and pyroligneous acids.

The form of the aromatic agent composition of the present invention is not particularly limited. The aromatic agent composition may be in a liquid, gel or other form. The aromatic agent is preferably in liquid form.

The aromatic agent composition of the present invention is prepared by mixing predetermined amounts of the fragrance material, the glycol ether, and, as necessary, other components.

The aromatic agent composition of the present invention is used as an aromatic agent placed in an openable storage space. In the present invention, "an openable storage space" refers to a storage space that can be opened and closed by the user to store the objects or take out the objects. Examples of the openable storage spaces include drawers, footwear cupboards, clothing storage, accessory cases, and cabinets. Among these storage spaces, footwear cupboards and drawers, particularly drawers, are suitable for the aromatic agent composition of the present invention. The capacity of the storage space where the aromatic agent composition of the present invention is placed is not particularly limited, and is generally in a range of from 0.5 to 150 L, preferably 1 to 100 L, further preferably 10 to 80 L. The effect of the aromatic agent composition of the present invention, i.e., the effect of maintaining a constant and homogeneous intensity of an aroma, is particularly effective for storage spaces of the above-specified capacities.

Further, the aromatic agent composition of the present invention also serves to transfer the aroma to the objects stored in the storage spaces. Therefore, by placing the aromatic agent composition of the present invention in a clothing storage space, the pleasant aroma is transferred to the clothing in the storage space. In view of this effect of the present invention, the aromatic agent composition of the present invention is suitable for closets or clothing storage spaces.

The aromatic agent composition of the present invention is used by being stored in a container that allows the fragrance material to spread outward. The structure of the container is determined according to the form of the aromatic agent composition. For example, for a liquid aromatic agent, it is preferable to use (i) a container having a component serving both as an absorber and an emitter that absorbs the aromatic agent composition in the container and emits the absorbed composition; or (ii) a container with a sealed opening comprising an existing permeable film that blocks the liquid aromatic agent composition therein while allowing the volatilized aromatic agent composition to permeate. The second (ii) container is particularly suitable to contain the aromatic agent composition of the present invention because it prevents the liquid from flowing out of the container even when the container is shaken by impact upon the opening or closing of the storage space. The liquid aromatic agent composition can be used by being impregnated in a core material made of paper, pulp, synthetic fiber, synthetic resin, and natural resins such as cellulose or the like. By impregnating the liquid aromatic composition in such a core material, it is possible to prevent the spill of liquid upon the opening or closing of the storage space.

### II. Process for emitting an aroma to openable storage spaces

As described above, the aromatic agent composition is capable of emitting an aroma to an openable storage space at a homogeneous and constant intensity. Accordingly, the present invention also provides a process for emitting an aroma to openable storage spaces; the process comprising emitting an aromatic agent composition containing a fragrance material, and 15 to 90 wt% of a glycol ether having a boiling point of 120 to 185°C under ordinary pressure, to an openable storage space.

In this method of the present invention, the constitution of the aromatic agent composition, the openable storage spaces where the aromatic agent composition is placed, and the usage of the aromatic agent composition are the same as those in the above section "I. Aromatic agent composition".

### III. Process for adjusting volatility of aroma in openable storage spaces

As described above, by mixing a fragrance material with a glycol ether having a boiling point of 120 to 185°C under ordinary pressure so that the glycol ether is present in an amount of 15 to 90 wt%, it is possible to adjust the volatility of the aroma, thereby providing aroma emission to a closed storage space at a homogeneous and constant intensity. Accordingly, the present invention provides a process for adjusting a volatility of an aroma emitted to an openable storage space; the process comprising mixing a fragrance material with a glycol ether having a boiling point of 120 to 185°C under ordinary pressure so that the proportion of the glycol ether in the resulting aromatic agent composition is 15 to 90 wt%.

In this adjustment method of the present invention, the types and proportions of the fragrance material and the glycol ether are the same as those in the above section "I. Aromatic agent composition".

### [Example 1]

### [Example]

The present invention is more specifically explained below in reference to Examples etc. The present invention is, however, not limited to these examples.

### Test Example 1

### Investigation of volatility characteristic of fragrance material in drawer

Nine aromatic agent compositions were prepared according to the constitutions shown in Table 1. For each composition, the volatility characteristic of the fragrance material in a drawer was observed.

**[Table 1]**

| Unit: wt% | | | | | | | | | | |
|---|---|---|---|---|---|---|---|---|---|---|
| Constitution | | Examples | | | | | | | | |
| | | 1 | 2 | 3 | 4 | 5 | 6 | 7 | 8 | 9 |
| Fragrance | d-limonene | 10 | 30 | 40 | | | | | | |
| | Caryophyllene | 10 | 30 | 40 | | | | | | |
| | cis-3-hexenol | | | | 20 | | | | | |
| | Geraniol | | | | 20 | | | | | |
| | Phenylethyl alcohol | | | | 20 | | | | | |
| | Diphenyloxide | | | | | 30 | | | | |
| | dl-roseoxide | | | | | 30 | | | | |
| | Hexanal | | | | | | 30 | | | |
| | Hexyl cinnamic aldehyde | | | | | | 30 | | | |
| | Ethyl butyrate | | | | | | | 10 | 20 | |
| | Styralyl acetate | | | | | | | 10 | 20 | |
| | o-t-butylcyclohexyl acetate | | | | | | | 10 | 20 | |
| | 2-octanone | | | | | | | | | 30 |
| | δ-damascone | | | | | | | | | 30 |
| Solvent | Monopropylene glycol mono-n-propyl ether^{#} | 80 | 40 | 20 | 40 | 40 | 40 | 70 | 40 | 40 |
| Total | | 100 | 100 | 100 | 100 | 100 | 100 | 100 | 100 | 100 |

| | | | | | | | | | | |
|---|---|---|---|---|---|---|---|---|---|---|
| ^{#}The monopropylene glycol mono-n-propyl ether used in each of the Examples is a product of Dow Chemical Japan Ltd. The monopropylene glycol mono-n-propyl ether has a boiling point of 150°C under normal pressure, and a vapor pressure of 0.205 kPa at 20°C. | | | | | | | | | | |

20 g each of the aromatic agent compositions prepared according to the constitutions shown in Table 1 was placed in a 40 ml container (area of the opening = about 1 cm²). Then, an absorbing/emitting member was inserted into a container containing an aromatic agent composition. The absorbing/emitting member had an absorbing portion for absorbing an aromatic agent composition from the container, and an emitting portion positioned outside the container for emitting the aromatic agent composition absorbed to the absorbing portion. The absorbing/emitting member was formed of pulp and rayon. The container ready for emitting the aromatic agent composition was placed in a drawer (wood, 50 × 40 × 8.5 cm). The drawer was completely closed, and the emission of the aromatic agent composition was started. The drawer was opened after 20 minutes, four days, and seven days of the emission, and 6 panelists evaluated the aroma according to the following evaluation criteria. During the investigation, the drawer was kept closed except for the evaluations by the panelists after 20 minutes, four days, and seven days of the emission of the aromatic agent composition.

### Evaluation Criteria

1 point: a weak aroma was perceived
2 points: a relatively weak aroma was perceived
3 points: an aroma of proper intensity was perceived
4 points: a relatively strong aroma was perceived
5 points: a strong aroma was perceived

Further, the concentrations of the fragrance material and the solvent in the aromatic agent composition in the container were measured using gas chromatography. The measurement was performed before the emission, and after four days and seven days of the emission.

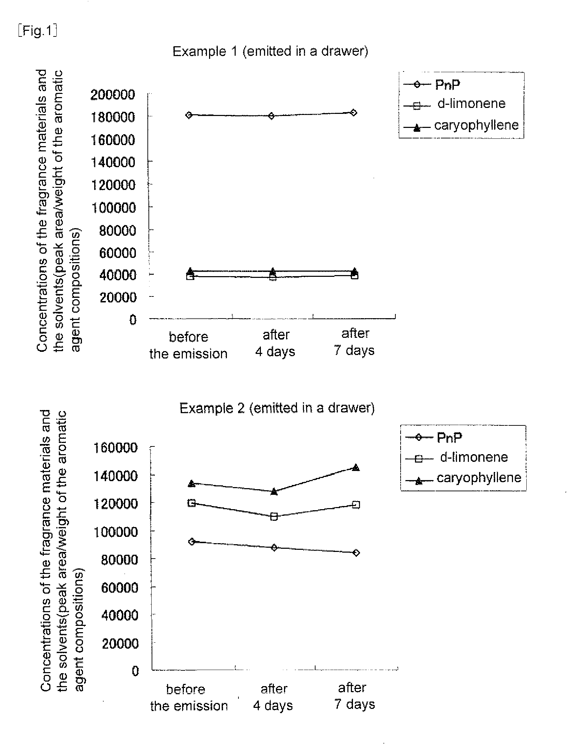

Table 2 shows the evaluation results of the aroma perception. FIG. 1 shows the measurement results of the concentrations of the fragrance material and the solvent in the aromatic agent composition. FIG. 1 shows the values of Example 1 and Example 2 as the representatives of the measurement results of the concentrations of the fragrance material and the solvent in the aromatic agent compositions of all the Examples. The results showed that the panelists perceived homogeneous aromas from the aromatic agent compositions of Examples 1 to 9 throughout the seven days of the emission. Further, the measurement results of the concentrations of the fragrance material and the solvent in the aromatic agent composition showed that the concentrations of the fragrance material and the solvent were substantially constant, confirming that the aromatic agent composition was capable of emitting an aroma at a homogeneous and constant intensity even in a drawer that is repeatedly opened and closed.

**[Table 2]**

| | Evaluation of Aroma^{#1} | | | Difference in Evaluation of Aroma^{#2} (after 20 minutes - after 7 days) |
|---|---|---|---|---|
| | After 20 minutes | After 4 days | After 7 days | |
| Example 1 | 2.1 | 1.9 | 1.6 | 0.5 |
| Example 2 | 2.7 | 2.7 | 2.6 | 0.1 |
| Example 3 | 2.4 | 2.3 | 2.1 | 0.3 |
| Example 4 | 3.4 | 3.4 | 3.2 | 0.2 |
| Example 5 | 3.6 | 3.4 | 3.0 | 0.6 |
| Example 6 | 4.0 | 3.8 | 3.5 | 0.5 |
| Example 7 | 2.6 | 2.4 | 2.2 | 0.4 |
| Example 8 | 3.7 | 3.5 | 3.4 | 0.3 |
| Example 9 | 3.3 | 3.1 | 2.8 | 0.5 |

| | | | | |
|---|---|---|---|---|
| ^{#}1 Average value of the evaluation results given by 6 panelists ^{#}2 A value obtained by subtracting the value after 7 days from the value after 20 minutes | | | | |

### Reference Test Example 1

### Investigation of volatility characteristic of fragrance material in a room

As in Test Example 1, nine aromatic agent compositions were prepared according to the constitutions shown in Table 1. Each composition was placed in a container to be ready for emission. The container was left to stand in a room, and emission of the aromatic agent composition was started. Before the aroma emission, and after four days and seven days of the emission, the container containing the aromatic agent composition was moved to a stainless storage container (capacity = 1,000 L), which was kept at 22°C and nearly free of scent and wind. After leaving the container to stand for 20 minutes, the perception of aroma was evaluated in the same manner as in Test Example 1. Further, the concentrations of the fragrance material and the solvent in the aromatic agent composition in the container were measured in the same manner as in Test Example 1, before the emission, and after four days and seven days of the emission.

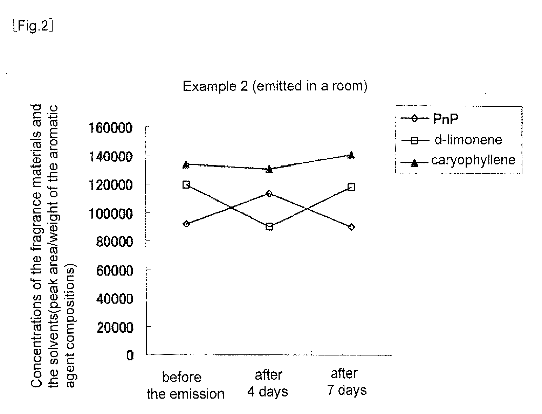

Table 3 shows the evaluation results of the aroma perception. FIG. 2 shows the measurement results of the concentrations of the fragrance material and the solvent in the aromatic agent composition. Table 3 shows the values of Examples 3, 5, 6 and 9 as the representatives of the aroma perception evaluation results. Thus, the evaluation results of the aroma perception for Examples 1, 2, 4 and 7 to 8 are omitted here; however, the values of Examples 1, 2, 4 and 7 to 8 were similar to the values of Examples 3, 5, 6 and 9. FIG. 2 only shows the value of Example 2 as a representative of the measurement results of the concentrations of the fragrance material and the solvent in the aromatic agent composition of all Examples. Thus, the measurement results of the aroma perception for Examples 1 and 3 to 9 are omitted here; however, the values of Examples 1 and 3 to 9 are similar to the value of Example 2.

The results showed that, for the aromatic agent compositions of Examples 1 to 9, the aroma perception became uneven with time when the compositions were placed in a room. The results also showed that the concentrations of the fragrance material and the solvent also changed with time.

**[Table 3]**

| | Evaluation of Aroma^{#1} | | | Difference in Evaluation of Aroma^{#2} (before emission - after 7 days) |
|---|---|---|---|---|
| | Before aroma emission | After 4 days | After 7 days | |
| Example 3 | 3.5 | 2.7 | 2.3 | 1.3 |
| Example 5 | 5.0 | 4.0 | 3.1 | 1.9 |
| Example 6 | 4.9 | 4.3 | 3.6 | 1.3 |
| Example 9 | 3.8 | 3.2 | 2.2 | 1.6 |

| | | | | |
|---|---|---|---|---|
| ^{#}1 Average value of evaluation results given by 6 panelists ^{#}2 Value obtained by subtracting the value after 7 days from the value before aroma emission | | | | |

The above results of Reference Test Example 1 showed that the aromatic agent compositions of Examples 1 to 9 are more suitable for closed storage spaces, such as drawers, than for general rooms.

### Reference Test Example 2

### Investigation of volatility characteristic of fragrance material mixed with other solvents

An aromatic agent composition was prepared using a solvent, other than the glycol ether, having a boiling point of 120 to 185°C under ordinary pressure; or 10 wt% of monopropylene glycol monoalkyl ether. With this aromatic agent composition, the volatility characteristic of the fragrance material in a drawer was evaluated. More specifically, seven aromatic agent compositions were prepared according to the constitutions shown in Table 4. In the same method as in Test Example 1, each aromatic agent composition was placed in a drawer, and the aroma emission was started. After 20 minutes, four days and seven days of the aroma emission, the drawer was opened and the aroma perception was evaluated in the same manner as in Test Example 1.

**[Table 4]**

| Unit: wt% | | | | | | | | |
|---|---|---|---|---|---|---|---|---|
| Constitution | | Comparative Examples | | | | | | |
| | | 1 | 2 | 3 | 4 | 5 | 6 | 7 |
| Fragrance | d-limonene | 30 | 30 | 30 | | | | |
| | Caryophyllene | 30 | 30 | 30 | | | | |
| | Ethyl butyrate | | | | 20 | 20 | 20 | 30 |
| | Styralyl acetate | | | | 20 | 20 | 20 | 30 |
| | o-t-butylcyclohexyl acetate | | | | 20 | 20 | 20 | 30 |
| solvent | Isopar MB | 40 | | | 40 | | | |
| | DPM | | 40 | | | 40 | | |
| | Solfit | | | 40 | | | 40 | |
| | Monopropylene glycol mono-n-propyl ether | | | | | | | 10 |
| Total | 100 | 100 | 100 | 100 | 100 | 100 | 100 | 100 |

| | | | | | | | | |
|---|---|---|---|---|---|---|---|---|
| ^{#}1 The Isopar MB used in each of the Examples is a product of Exxon Mobil Corporation. The Isopar MB is an isoparaffin hydrocarbon having a boiling point of 225 to 254°C under normal pressure, and a vapor pressure of 0.01 kPa at 20°C. ^{#}2 The DOWANOL DPM used in each of the Examples is a product of Dow Chemical Japan Ltd. The DOWANOL DPM is a dipropylene glycol monoethyl ether having a boiling point of 190°C under normal pressure, and a vapor pressure of 0.037 kPa at 20°C. ^{#}3 The Solfit used in each of the Examples is a product of Kuraray Co. Ltd. The Solfit is a 3-methoxy-3-methyl-1-butanol having a boiling point of 174°C under normal pressure. ^{#}4 The monopropylene glycol mono-n-propyl ether used in each of the Examples is a product of Dow Chemical Japan Ltd. The monopropylene glycol mono-n-propyl ether has a boiling point of 150°C under normal pressure, and a vapor pressure of 0.205 kPa at 20°C. | | | | | | | | |

The results showed that the intensity in the aroma perception apparently changed after seven days of the aroma emission, compared with the intensity perceived after 20 minutes of the aroma emission. This confirmed that the aroma perception became uneven with time.

The above results showed that the aroma emission in a storage space can be kept at a homogeneous and constant intensity by using an aromatic agent composition obtained by mixing a fragrance material (10 to 85 wt%, preferably 30 to 80 wt%) with a glycol ether having a boiling point of 120 to 185°C under ordinary pressure, that is present in an amount of 15 to 90 wt%, more preferably, 20 to 70 wt%. The results also showed that, when the fragrance material is mixed with other solvents, the aroma emission in a storage space such as a drawer becomes uneven with time; therefore, the desired comfortable space filled with a pleasant aroma cannot be obtained with the use of other solvents.

### [Brief Description of Drawings]

FIG. 1 shows the measurement results of the concentrations of the fragrance materials and the solvents after the aromatic agent compositions of Example 1 and Example 2 were emitted in a drawer. In the figures, PnP stands for monopropylene glycol mono-n-propyl ether. The upper portion of FIG. 1 shows the data of the aromatic agent composition of Example 1, and the lower portion of FIG. 1 shows the data of the aromatic agent composition of Example 2.
FIG. 2 shows the measurement results of the concentrations of the fragrance material and the solvent after the aromatic agent compositions of Example 2 was emitted in a room. PnP stands for monopropylene glycol mono-n-propyl ether.

## Claims

1. An aromatic agent composition for an openable storage space, comprising a fragrance material and 15 to 90 wt% of a glycol ether having a boiling point of 120 to 185°C under ordinary pressure.

2. The aromatic agent composition according to claim 1, wherein the glycol ether is present in an amount of 25 to 55 wt% of the glycol ether.

3. The aromatic agent composition according to claim 1, wherein the glycol ether has a vapor pressure of 0.05 to 1 kPa at 20°C.

4. The aromatic agent composition according to claim 1, wherein the glycol ether is a monopropylene glycol monoalkyl ether.

5. The aromatic agent composition according to claim 1, wherein the glycol ether is a monopropylene glycol mono-n-propyl ether.

6. The aromatic agent composition according to claim 1, wherein the fragrance material is present in an amount of 10 to 85 wt%.

7. The aromatic agent composition according to claim 1, wherein the fragrance material has a boiling point of 50 to 360°C under ordinary pressure.

8. The aromatic agent composition according to claim 1, wherein the fragrance material is at least one member selected from the group consisting of d-limonene, caryophyllene, cis-3-hexenol, geraniol, phenylethyl alcohol, diphenyloxide, dl-roseoxide, hexanal, hexyl cinnamic aldehyde, ethyl butyrate, styralyl acetate, o-t-butylcyclohexyl acetate, 2-octanone and δ-damascone.

9. The aromatic agent composition according to claim 1, wherein the fragrance material is present in an amount of 45 to 75 wt%, and the glycol ether is present in an amount of 25 to 55 wt%.

10. The aromatic agent composition according to claim 1, wherein the aromatic agent composition is a liquid.

11. The aromatic agent composition according to claim 1, wherein the aromatic agent composition is placed in a drawer or a footwear cupboard.

12. The aromatic agent composition according to claim 1, wherein the aromatic agent composition is placed in a clothing storage space.

13. A process for producing an aromatic agent composition for an openable storage space, comprising:
mixing a fragrance material and a glycol ether having a boiling point of 120 to 185°C under ordinary pressure so that the glycol ether is present in an amount of 15 to 90 wt%.

14. A process for emitting an aroma to an openable storage space, comprising:
emitting an aromatic agent composition comprising a fragrance material and 15 to 90 wt% of a glycol ether having a boiling point of 120 to 185°C under ordinary pressure, to an openable storage space.

15. A process for adjusting a volatility of an aroma emitted to an openable storage space, comprising:
mixing a fragrance material and a glycol ether having a boiling point of 120 to 185°C under ordinary pressure so that the glycol ether is present in an amount of 15 to 90 wt%.

16. Use of a composition comprising a fragrance material and 15 to 90 wt% of a glycol ether having a boiling point of 120 to 185°C under ordinary pressure, for producing an aromatic agent composition for an openable storage space.

17. Use of a glycol ether having a boiling point of 120 to 185°C under ordinary pressure, for adjusting a volatility of a fragrance material in an openable storage space, the glycol ether being present in an amount of 15 to 90 wt% with respect to a gross amount of an aromatic agent composition.
